# EUROPEAN PATENT APPLICATION

(11) **EP 4 458 355 A1**
(43) Date of publication of application: **06.11.2024**
(21) Application number: 22913206.3
(22) Date of filing: 22.06.2022
(51) Int. Cl.: A61K 31/4035, C12N 9/99, C12N 9/22

(54) **TAQ ENZYME REVERSIBLE MODIFIER, CHEMICALLY MODIFIED TAQ ENZYME, AND PREPARATION METHOD THEREFOR AND PCR KIT THEREOF**

(30) Priority: 30.12.2021 CN 202111658616
(71) Applicant: Daan Gene Co., Ltd., Guangzhou, Guangdong 510665 (CN)
(72) Inventor: HE, Ling, Guangzhou, Guangdong 510665 (CN); JIANG, Xiwen, Guangzhou, Guangdong 510665 (CN); HUANG, Jianfeng, Guangzhou, Guangdong 510665 (CN); NIE, Jianhong, Guangzhou, Guangdong 510665 (CN); PAN, Xufeng, Guangzhou, Guangdong 510665 (CN)
(74) Representative: Diehl & Partner
(86) International application number: PCT/CN2022/100277
(87) International publication number: WO 2023/123917

(57) **Abstract**

A Taq enzyme reversible modifier, a chemically modified Taq enzyme, and a preparation method therefor and a PCR kit thereof. The structure of the Taq enzyme reversible modifier is as shown in formula (1). The activity of the Taq enzyme modified with the Taq enzyme reversible modifier is better than the activity of the Taq enzyme modified with N-ethoxycarbonyl-2,3-disubstituted maleimide.

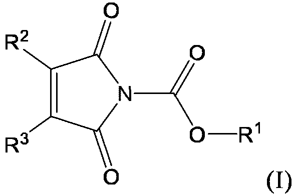

## Description

### Cross-reference to related application

This patent application claims priority to Chinese patent application submitted on December 30, 2021, with the application number 202111658616.4 and the invention name *"Taq enzyme reversible modifier, chemically modified Taq enzyme and preparation method therefor and PCR kit thereof",* The entire text of the above application is incorporated herein by reference.

### Technical Field

The invention relates to the field of instant detection, and in particular to Taq enzyme reversible modifier, chemically modified Taq enzyme and preparation method therefor and PCR kit thereof.

### Background

Taq enzyme is the most commonly used tool enzyme for PCR, but because it is still partially active at room temperature, non-specific amplification products often occur, especially when there is a large amount of non-specific DNA in the PCR reaction system. At present, chemical modification methods are often used to modify Taq enzyme. That is reacting with Taq enzyme using a Taq enzyme reversible modifier. The activity of the modified hot-start enzyme can be inhibited at room temperature and it can quickly recover its high activity at high temperature, thus avoiding non-specific amplification at room temperature.

In the prior art, dicarboxylic anhydride is often used as a Taq enzyme reversible modifier to modify Taq enzyme. In addition, patent US2007224611A1 discloses the compound of the following formula 3 (N-ethoxycarbonyl-2,3-disubstituted butenedimide) as a reversible modifier for thermophilic enzymes, and discloses its preparation and purification methods.

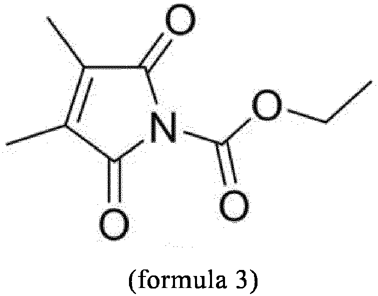

However, the inventor found through research that the Taq enzyme reversible modifier prepared and purified by the above method modified the Taq enzyme resulted in poor activity. Therefore, there is still a need in this field to find Taq enzyme reversible modifiers with better modification effects and methods to improve the modification effect of Taq enzyme reversible modifiers.

### Summary of the invention

The purpose of the present invention is to provide a Taq enzyme reversible modifier.

Another purpose of the present invention is to provide a Taq enzyme modified by the above-mentioned Taq enzyme reversible modifier.

Another purpose of the present invention is to provide a PCR kit containing the above-mentioned Taq enzyme modified by the Taq enzyme reversible modifier.

Another purpose of the present invention is to provide a method for improving the activity of the above-mentioned Taq enzyme modified by the Taq enzyme reversible modifier.

In order to solve the above technical problems, the first aspect of the present invention provides a Taq enzyme reversible modifier, wherein the structure of the Taq enzyme reversible modifier is shown in formula (1) 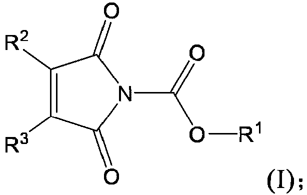
wherein, R¹ is C₃₋₆ alkyl;
R² and R³ are independently C₁₋₆ alkyl, respectively.

In some preferred embodiments, R¹ is unsubstituted C₃₋₆ linear alkyl;
and/or, R² and R³ are independently C₁₋₄ alkyl, respectively.

In some preferred embodiments, R¹ is n-propyl, n-butyl, n-pentyl or n-hexyl.

In some preferred embodiments, R² and R³ are independently methyl, ethyl, n-propyl, isopropyl, n-butyl, sec-butyl, isobutyl or tert-butyl, respectively.

In some preferred embodiments, R¹ is n-hexyl.

In some preferred embodiments, the structure of the Taq enzyme reversible modifier is shown in formula (II):

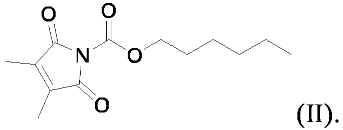

In some preferred embodiments, the Taq enzyme reversible modifier is obtained by purifying the crude product of 3,4-dimethyl-2,5-dicarbonyl-2,5-dihydro-1H-pyrrole-1-carboxylic acid hexyl ester using high performance liquid chromatography.

In the second aspect of the present invention, it provides a chemically modified Taq enzyme, which is modified by the Taq enzyme reversible modifier described in the first aspect of the present invention.

In the third aspect of the present invention, it provides a PCR kit, which comprises the chemically modified Taq enzyme described in the second aspect of the present invention.

In the fourth aspect of the present invention, it provides a method for preparing the chemically modified Taq enzyme provided in the third aspect of the present invention, the method includes the step of mixing wild-type Taq enzyme and the Taq enzyme reversible modifier described in the first aspect of the present invention.

In some preferred embodiments, the mixing is performed in an oscillator, and the mixing time is 15 to 30 seconds.

In the fifth aspect of the present invention, it provides a method for improving the activity of chemically modified Taq enzyme, wherein the method includes the steps: the prepared crude product solution of the chemical modifier is purified using high performance liquid chromatography, and then subjected to a modification reaction with the Taq enzyme to be modified.

In some preferred embodiments, the chemical modifier is a compound represented by formula (II)

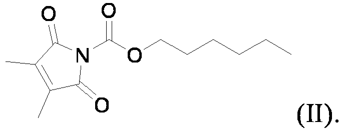

In some preferred embodiments, the method includes the steps of:
(1) purifying the crude product solution of the compound represented by formula (II) using preparative high performance liquid chromatography and collecting target fraction; and
(2) extracting the target fraction obtained in step (1) and then rotary evaporating to obtain the purified compound represented by formula (I); and
(3) subjecting the purified compound represented by formula (II) obtained in step (2) to a modification reaction with the Taq enzyme to be modified.

In some preferred embodiments, in step (1), the chromatographic column used in the preparative high performance liquid chromatography method is a C18 chromatographic column.

In some preferred embodiments, the mobile phase used in the preparative high performance liquid chromatography method is a mixed solution of water and acetonitrile.

In some preferred embodiments, the preparative high performance liquid chromatography method is performed using gradient elution.

In some preferred embodiments, the flow rate of mobile phase is 3.5 to 4.5 mL/minute, such as 4 mL/minute.

In some preferred embodiments, the particle size of the C18 chromatography column filler is 2 µm to 10 µm, such as 2 µm, 5 µm or 10 µm.

In some preferred embodiments, the gradient elution procedure is: within the time period t, the volume percentage of acetonitrile in the mobile phase is changed from 10% to 100% at a constant speed; the t is 25 to 35 minutes, such as 30 minutes.

In some preferred embodiments, the target fraction is an eluate with a retention time of 15 to 19 minutes, for example, an eluate with a retention time of 17 to 18 minutes.

In some preferred embodiments, in step (2), the extraction agent used in the extraction is methylene chloride.

In some preferred embodiments, in step (3), the modification reaction includes the step of mixing the purified compound represented by formula (II) obtained in step (2) with the Taq enzyme to be modified.

In some preferred embodiments, the mixing is performed using an oscillator, and the oscillation time of the oscillator is 15 to 30 seconds, such as 20 seconds.

In the sixth aspect of the present invention, it provides the use of a compound of formula (I) for:
(i) preparing Taq enzyme reversible modifier;
(ii) modifying Taq enzyme reversibly.

Compared with the prior art, the embodiments of the present invention at least have the following beneficial effects:
(1) the activity of Taq enzyme modified by the Taq enzyme reversible modifier provided by the present invention is better than that of Taq enzyme modified with N-ethoxycarbonyl-2,3-disubstituted butenedimide;
(2) the stability of the chemically modified Taq enzyme provided by the present invention is high;
(3) the method for improving the activity of chemically modified Taq enzyme provided by the present invention can greatly improve the activity of chemically modified Taq enzyme in a low-cost and simple way, especially the activity of Taq enzyme modified by the compound represented by formula (I) or formula (II).

It should be understood that, within the scope of the present invention, the above-mentioned technical features of the present invention and the technical features specifically described below (such as embodiments) can be combined with each other to form new or preferred technical solutions. Due to the limitation of space, they will not be described one by one here.

### Description of drawings

One or more embodiments are exemplarily illustrated by the figures in the corresponding drawings, which do not constitute a limitation of the embodiments.
Figure 1 is an HPLC diagram of pure 3,4-dimethyl-2,5-dioxo-2,5-dihydro-1H-pyrrole-1-carboxylic acid hexyl ester in an embodiment of the present invention;
Figure 2 is an LC-MS spectrum of pure 3,4-dimethyl-2,5-dioxo-2,5-dihydro-1H-pyrrole-1-carboxylic acid hexyl ester in an embodiment of the present invention;
Figure 3 is a ¹H MNR spectrum of 3,4-dimethyl-2,5-dioxo-2,5-dihydro-1H-pyrrole-1-carboxylic acid hexyl ester in an embodiment of the present invention;
Figure 4 is a graph of melting curve of enzyme activity with DNA polymerase modified with 3,4-dimethyl-2,5-dicarbonyl-2,5-dihydro-1H-pyrrole-1-carboxylic acid hexyl ester, wild-type DNA polymerase and without DNA polymerase in an embodiment of the present invention.
Figure 5 is a diagram showing the enzyme activity test results of the 0.8x experimental group and the control group according to an embodiment of the present invention;
Figure 6 is a diagram showing the enzyme activity test results of the 1.0x experimental group and the control group according to an embodiment of the present invention;
Figure 7 is a diagram showing the enzyme activity test results of the 1.2x experimental group and the control group according to an embodiment of the present invention;
Figure 8 is a graph showing accelerated stability results of the 0.8x experimental group and the control group according to an embodiment of the present invention;
Figure 9 is a graph showing accelerated stability results of the 1.0x experimental group and the control group according to an embodiment of the present invention;
Figure 10 is a graph showing accelerated stability results of the 1.2x experimental group and the control group according to an embodiment of the present invention.

### Detailed description of the invention

Through detailed research, the inventor found that the activity of the Taq enzyme modified by the butenedimide modifier (such as formula A below) is significantly better than that of the commonly used dicarboxylic anhydride-modified Taq enzyme. In butenedimide modifiers, the N-terminal substituent R¹ significantly affects the modification effect. For example, in the test examples of the present invention, it was verified that the activity of Taq enzyme modified with 3,4-dimethyl-2,5-dicarbonyl-2,5-dihydro-1H-pyrrole-1-carboxylic acid hexyl ester was significantly better than Taq enzyme modified with N-ethoxycarbonyl-2,3-disubstituted butenedimide. 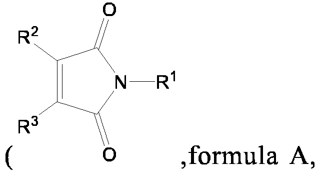 wherein R¹, R² and R³ are independently selected from alkyl, alkenyl, alkynyl, cycloalkyl, hydroxyl, alkoxy, halogen, amine, amino, nitro, amide or acyloxy, respectively.)

The activity of Taq enzyme modified with 3,4-dimethyl-2,5-dicarbonyl-2,5-dihydro-1H-pyrrole-1-carboxylic acid hexyl ester is significantly better than that of Taq enzyme modified with dicarboxylic anhydride or N-ethoxycarbonyl - 2,3-disubstituted butenedimide.

The Taq enzyme modified by the butenedimide modifier treated by the method for improving the activity of chemically modified Taq enzyme according to the present invention has better enzyme activity and high stability. Preferably, the Taq enzyme modified by 3,4-dimethyl-2,5-dicarbonyl-2,5-dihydro-1H-pyrrole-1-carboxylic acid hexyl ester treated by the method for improving the activity of chemically modified Taq enzyme according to the present invention was significantly higher than that of Taq enzyme modified with untreated 3,4-dimethyl-2,5-dicarbonyl-2,5-dihydro-1H-pyrrole-1-carboxylic acid hexyl ester.

Some embodiments of the present invention provide a Taq enzyme reversible modifier, and the structure of the Taq enzyme reversible modifier is shown in formula (I) 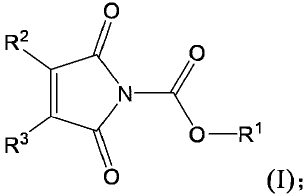
wherein, R¹ is C₃₋₆ alkyl;
R² and R³ are independently C₁₋₆ alkyl, respectively.

In some preferred embodiments, R¹ is unsubstituted C₃₋₆ linear alkyl;
and/or, R² and R³ are independently C₁₋₄ alkyl, respectively.

In some preferred embodiments, R¹ is n-propyl, n-butyl, n-pentyl or n-hexyl.

In some preferred embodiments, R² and R³ are independently methyl, ethyl, n-propyl, isopropyl, n-butyl, sec-butyl, isobutyl or tert-butyl, respectively.

In some preferred embodiments, R¹ is n-hexyl.

In some preferred embodiments, the structure of the Taq enzyme reversible modifier is shown in formula (II):

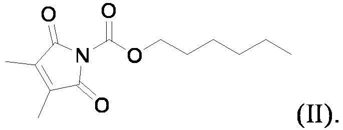

In some preferred embodiments, the Taq enzyme reversible modifier is obtained by purifying the crude product of 3,4-dimethyl-2,5-dicarbonyl-2,5-dihydro-1H-pyrrole-1-carboxylic acid hexyl ester using high performance liquid chromatography.

Some embodiments of the present invention provide a chemically modified Taq enzyme, which is modified by the Taq enzyme reversible modifier described in the first aspect of the present invention.

Some embodiments of the present invention provide a PCR kit, which includes the chemically modified Taq enzyme described in the second aspect of the present invention.

Some embodiments of the present invention provide a method for preparing the chemically modified Taq enzyme provided in the third aspect of the present invention, the method includes the step of mixing wild-type Taq enzyme and the Taq enzyme reversible modifier described in the first aspect of the present invention.

In some preferred embodiments, the mixing is performed in an oscillator, and the mixing time is 15 to 30 seconds.

Some embodiments of the present invention provide a method for improving the activity of chemically modified Taq enzyme, wherein the method includes the steps: the prepared crude product solution of the chemical modifier is purified using high performance liquid chromatography, and then subjected to a modification reaction with the Taq enzyme to be modified.

In some preferred embodiments, the chemical modifier is a compound represented by formula (II)

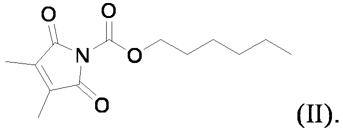

In some preferred embodiments, the method includes the steps of:
(1) purifying the crude product solution of the compound represented by formula (II) using preparative high performance liquid chromatography and collecting the target fraction; and
(2) extracting the target fraction obtained in step (1) and then rotary evaporating to obtain the purified compound represented by formula (I); and
(3) subjecting the purified compound represented by formula (II) obtained in step (2) to a modification reaction with the Taq enzyme to be modified.

In some preferred embodiments, in step (1), the chromatographic column used in the preparative high performance liquid chromatography method is a C18 chromatographic column.

In some preferred embodiments, the mobile phase used in the preparative high performance liquid chromatography method is a mixed solution of water and acetonitrile.

In some preferred embodiments, the preparative high performance liquid chromatography method is performed using gradient elution.

In some preferred embodiments, the flow rate of mobile phase is 3.5 to 4.5 mL/minute, such as 4 mL/minute.

In some preferred embodiments, the particle size of the C18 chromatography column filler is 2 µm to 10 µm, such as 2 µm, 5 µm or 10 µm.

In some preferred embodiments, the gradient elution procedure is: within the time period t, the volume percentage of acetonitrile in the mobile phase is changed from 10% to 100% at a constant speed; the t is 25 to 35 minutes, such as 30 minutes.

In some preferred embodiments, the target fraction is an eluate with a retention time of 15 to 19 minutes, for example, an eluate with a retention time of 17 to 18 minutes.

In some preferred embodiments, in step (2), the extraction agent used in the extraction is methylene chloride.

In some preferred embodiments, in step (3), the modification reaction includes the step of mixing the purified compound represented by formula (II) obtained in step (2) with the Taq enzyme to be modified.

In some preferred embodiments, the mixing is performed using an oscillator, and the oscillation time of the oscillator is 15 to 30 seconds, such as 20 seconds.

Some embodiments of the present invention provide the use of a compound of formula (I) for:
(i) preparing Taq enzyme reversible modifier;
(ii) modifying Taq enzyme reversibly.

In order to make the purpose, technical solutions and advantages of the embodiments of the present invention clearer, the present invention will be further described below in combination with specific embodiments. It should be understood that these examples are only used to illustrate the invention and are not intended to limit the scope of the invention. The experimental methods for which specific conditions are not indicated in the following examples are generally in accordance with conventional conditions or in accordance with the conditions recommended by the manufacturer. Unless otherwise indicated, all percentage and parts are calculated by weight. The experimental materials and reagents used in the following examples can be obtained from commercial sources unless otherwise specified.

Unless otherwise defined, technical and scientific terms used herein have the same meanings as generally understood by one of ordinary skill in the art to which this application belongs. It should be noted that the terms used herein are only for describing specific embodiments and are exemplary embodiments which are not intended to limit the present application.

### Example 1. Preparation of 3,4-dimethyl-2,5-dicarbonyl-2,5-dihydro-1H-pyrrole-1-carboxylic acid hexyl ester

At room temperature, 77.6 g of 1,1,1,3,3,3-hexamethyldisilazane was added to a solution of 1L N, N-dimethylformamide in which 30.5g of 2,3-dimethylmaleic anhydride was dissolved. The mixture was heated to 100°C and stirred for 30 min. Then it was lowered to room temperature, 3L of water was added to quench the reaction, and the resulting solution was extracted with 10L of ethyl acetate. The combined organic extracts were washed with 3L of brine, dried over Na2SO4, filtered, and concentrated in vacuo to obtain a crude residue. The residue was purified by silica gel column chromatography (eluent: hexane/AcOEt=3/1) to obtain 23.3g of crude 2,3-dimethylmaleimide as a white solid, which was set aside for use. At 0°C, 34.8g triethylamine and 42.5g n-hexyl chloroformate were added to 170ml of anhydrous dichloromethane solution in which 23.3 g of crude 2,3-dimethylmaleimide was dissolved. After the addition was completed, the temperature was raised to room temperature and the mixture was stirred for 20 minutes. The reaction was quenched by adding 3L of saturated aqueous NH₄Cl and the resulting solution was extracted with 10L of ethyl acetate. The combined organic extracts were washed with 3L of brine, dried over Na₂SO₄, filtered, and concentrated in vacuo to obtain 3,4-dimethyl-2,5-dicarbonyl-2,5-dihydro-1H-pyrrole-1-carboxylic acid hexyl ester crude product.

### Example 2: Purification of 3,4-dimethyl-2,5-dicarbonyl-2,5-dihydro-1H-pyrrole-1-carboxylic acid hexyl ester

### Purification way 1:

The crude product of 3,4-dimethyl-2,5-dioxo-2,5-dihydro-1H-pyrrole-1-carboxylic acid hexyl ester was dissolved in acetonitrile aqueous solution, and the resulting solution was filtered through a 0.45 µm microporous membrane. C18 with a filler particle size of 2 µm was used as the stationary phase. The system was equilibrated with a mobile phase system of water: acetonitrile = 90: 10 for 5 minutes. Adding the sample, start receiving the target fraction after elution for about 7 minutes, and receiving the target fraction for about 1 minute. The collected target fractions were extracted with dichloromethane, and the organic phase was collected and desalted. The desalted organic phase was treated with a rotary evaporator to obtain a colorless and transparent liquid, which is the pure product of 3,4-dimethyl-2,5-dioxo-2,5-dihydro-1H-pyrrole-1-carboxylic acid hexyl ester. After HPLC detection, the purity was 99.4%.

HPLC analysis and detection methods are as follows:

| | |
|---|---|
| Instrument | Waters e2695 analytical liquid phase |
| Chromatographic column | SunFire C18 100Å, 5 µm, 10 mmX250 mm |
| Injection volume | 100µL |
| Flow rate | 4Ml/min |
| Running time | 30min |
| Detection wavelength | 254nm |
| Mobile phase | Acetonitrile, water |
| Gradient | Acetonitrile content gradient changes from 10% to 100% within 0min~30min |

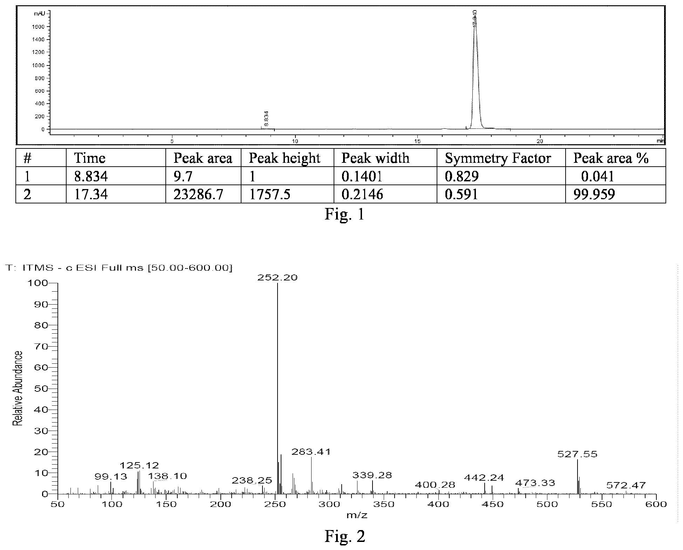

The chromatogram obtained by the above HPLC is shown in Figure 1.

The LC-MS spectrum of purified 3,4-dimethyl-2,5-dioxo-2,5-dihydro-1H-pyrrole-1-carboxylic acid hexyl ester is shown in Figure 2

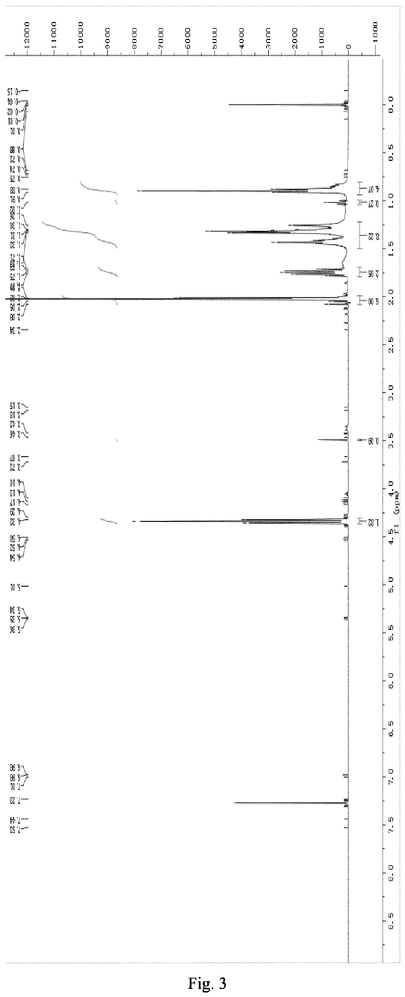

The ¹H MNR spectrum of purified 3,4-dimethyl-2,5-dioxo-2,5-dihydro-1H-pyrrole-1-carboxylic acid hexyl ester is shown in Figure 3.

### Purification way 2:

The crude product of 3,4-dimethyl-2,5-dioxo-2,5-dihydro-1H-pyrrole-1-carboxylic acid hexyl ester in Example 1 was dissolved in an acetonitrile/water solution, and the resulting solution was filtered through a 0.45 µm microporous membrane. C18 with a filler particle size of 5 µm was used as the stationary phase. The system was equilibrated with a mobile phase system of water: acetonitrile = 90:10 for 15 minutes. Adding the sample, start receiving the target fraction after elution for about 15 minutes, and receiving the target fraction for about 2 minutes. The collected target fractions were extracted with dichloromethane, and the organic phase was collected and desalted. The desalted organic phase was treated with a rotary evaporator to obtain a colorless and transparent liquid, which is the pure product of 3,4-dimethyl-2,5-dioxo-2,5-dihydro-1H-pyrrole-1-carboxylic acid hexyl ester. After detection, the purity was 99.2%.

HPLC analysis and detection methods are as follows:

| | |
|---|---|
| Instrument | Waters e2695 analytical liquid phase |
| Chromatographic column | SunFire C18 100Å, 5 µm, 10 mmX250 mm |
| Injection volume | 100µL |
| Flow rate | 4mL/min |
| Running time | 30min |
| Detection wavelength | 254nm |
| Mobile phase | Acetonitrile, water |
| Gradient | Acetonitrile content gradient changes from 10% to 100% within 0min~30min |

### Purification way 3:

The crude product of 3,4-dimethyl-2,5-dioxo-2,5-dihydro-1H-pyrrole-1-carboxylic acid hexyl ester was dissolved in 20% acetonitrile/water solution, and the resulting solution was filtered through a 0.45 µm microporous membrane. C18 with a filler particle size of 10 µm was used as the stationary phase. The system was equilibrated with a mobile phase system of water: acetonitrile = 90:10 for 20 minutes. Adding the sample, start receiving the target fraction after elution for about 35 minutes, and receiving the target fraction for about 5 minutes. The collected target fractions were extracted with dichloromethane, and the organic phase was collected and desalted. The desalted organic phase was treated with a rotary evaporator to obtain a colorless and transparent liquid, which is the pure product of 3,4-dimethyl-2,5-dioxo-2,5-dihydro-1H-pyrrole-1-carboxylic acid hexyl ester. After detection, the purity was 99.1%.

HPLC analysis and detection methods are as follows:

| | |
|---|---|
| Instrument | Waters e2695 analytical liquid phase |
| Chromatographic column | SunFire C18 100Å, 5 µm, 10 mmX250 mm |
| Injection volume | 100µL |
| Flow rate | 4mL/min |
| Running time | 30min |
| Detection wavelength | 254nm |
| Mobile phase | Acetonitrile, water |
| Gradient | Acetonitrile content gradient changes from 10% to 100% within 0min~30min |

### Example 3. Modification of Taq enzyme using crude and refined products of 3,4-dimethyl-2,5-dicarbonyl-2,5-dihydro-1H-pyrrole-1-carboxylic acid hexyl ester

Take the crude product of 3,4-dimethyl-2,5-dicarbonyl-2,5-dihydro-1H-pyrrole-1-carboxylic acid hexyl ester and mix it with wild-type DNA polymerase, and use an oscillator to shake for 20 seconds to obtain a DNA polymerase modified by the crude product of 3,4-dimethyl-2,5-dicarbonyl-2,5-dihydro-1H-pyrrole-1-carboxylic acid hexyl ester.

Replace crude product of 3,4-dimethyl-2,5-dicarbonyl-2,5-dihydro-1H-pyrrole-1-carboxylic acid hexyl ester with pure product of 3,4-dimethyl-2,5-dicarbonyl-2,5-dihydro-1H-pyrrole-1-carboxylic acid hexyl ester purified by Purification way 1,2,3 in example 2 to obtain a DNA polymerase modified with pure product of 3,4-dimethyl-2,5-dicarbonyl-2,5-dihydro-1H-pyrrole-1-carboxylic acid hexyl ester.

### Comparative Example 1: Modification of Taq enzyme with N-ethoxycarbonyl-2,3-disubstituted butenedimide

Mix N-ethoxycarbonyl-2,3-disubstituted butenedimide with wild-type DNA polymerase and use an oscillator to shake for 20 seconds to obtain a DNA polymerase modified with N-ethoxycarbonyl-2,3-disubstituted butenedimide.

### Example 4. Preparation of PCR reaction system and PCR experimental method

DNA polymerase activity test of the Taq DNA polymerase to be tested was performed using human single-stranded nucleic acid fragments as templates. The specific steps are as follows:

### (1) Design and synthesis of primers and templates

Referring to the chromosome 16 gene sequence registered in GenBank, design a single-stranded nucleic acid sequence and a primer complementary to the single-stranded nucleic acid sequence:

Single-stranded nucleic acid sequence:

GAGCCCGCCGCCCGGCCCCGCGCAGGCCCCGCCCGGGACTCCCCTGC
GGTCCAGGCCGCGCCCCGGGCTCCGCGCCAGCCAATGAGCGCCGCCCGG
CCGGGCGTGCCCCCGCGCCCCAAGCATAAACCCTGGCGCGCTCGCGGGCC
GGCACTCTTCTGGTCCCCACAGACTC（SEQ ID NO：1）

Primer sequence: 5'-GAGTCTGTGGGGAG-3' (SEQ ID NO: 2), both are artificially synthesized.

### (2) treatment of template and primer before PCR reaction

Dilute the synthesized templates and primers to 50 pmol/ul with sterile purified water or 1*TE (pH8.0) respectively, and store them for later use.

The optimal concentration of magnesium ions is 5mmol/L.

The optimal dosage of hot-start DNA polymerase (or hot-start DNA polymerase modification product) is 5U/reaction. The optimal dosage of UNG enzyme is 0.1U/reaction. The optimal concentration of deoxyribonucleoside triphosphates (dNTPs) is 0.24mmol/L.

### (3) Preparation of PCR reaction system

Take a PCR reaction tube and add 5 µL of 10x PCR buffer. Add magnesium chloride to make the concentration of working solution 5mmol/L. Add dNTPs to make the concentration of the working solution 0.24mmol/L. Add fluorescent dye to make the concentration of the working solution 1%. Add template to make the concentration of the working solution 50 pmol/ul. Add primers to make the concentration of the working solution 5pmol/ul. Add the DNA polymerase to be detected (or DNA polymerase modified with 3,4-dimethyl-2,5-dicarbonyl-2,5-dihydro-1H-pyrrole-1-carboxylic acid hexyl ester) to make the concentration of the working solution 2.5U, and then add purified water to make the total volume 50µL.

### (4) PCR reaction

Put each system prepared in the above step (3) into a fluorescence quantitative PCR instrument for reaction. The reaction conditions are: incubation at 37°C for 60 minutes.

### Test example 1. Blocking activity test of DNA polymerase

DNA polymerase was modified with purified 3,4-dimethyl-2,5-dicarbonyl-2,5-dihydro-1H-pyrrole-1-carboxylic acid hexyl ester.And the blocking activity of 3,4-dimethyl-2,5-dicarbonyl-2,5-dihydro-1H-pyrrole-1-carboxylic acid hexyl ester on DNA polymerase was measured. The specific method is as follows:

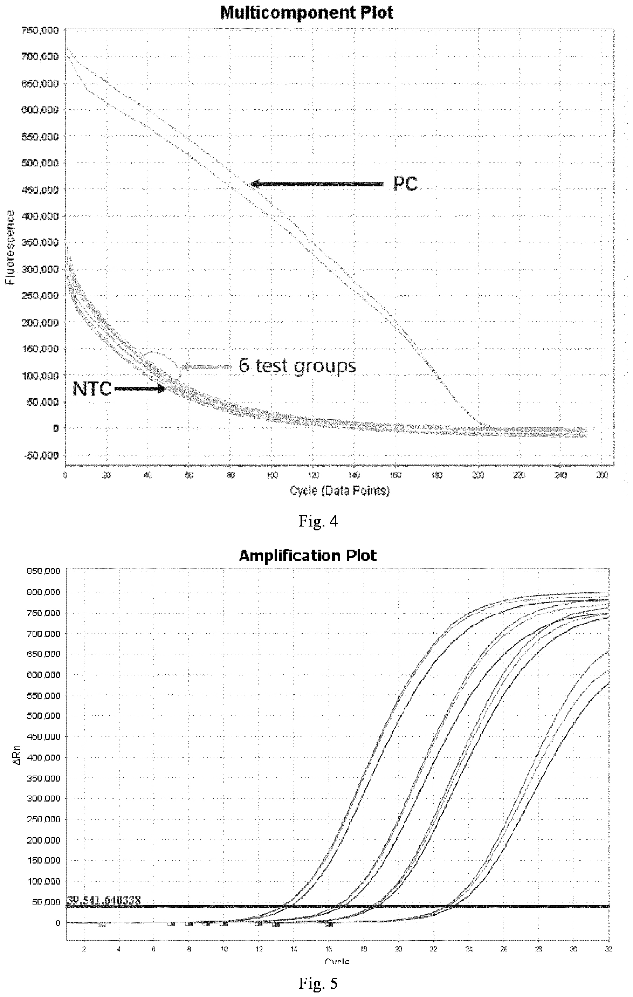

Add partially complementary primers to the qPCR system, and the purified 3,4-dimethyl-2,5-dicarbonyl-2,5-dihydro-1H-pyrrole-1-carboxylic acid hexyl ester-modified DNA polymerase enzyme was added thereto. Unmodified DNA polymerase naked enzyme was used as a positive control, and no enzyme at all was used as a negative control. Incubate at 37°C for 60 minutes to obtain the melting curve of the naked enzyme activity test, as shown in Figure 4.

In Figure 4, NTC is a system without enzyme, and PC is a naked enzyme system. It can be seen from Figure 4 that the enzyme curve after blocking with 3,4-dimethyl-2,5-dicarbonyl-2,5-dihydro-1H-pyrrole-1-carboxylic acid hexyl ester basically coincides with the curve without enzyme. It is visible that 3,4-dimethyl-2,5-dicarbonyl-2,5-dihydro-1H-pyrrole-1-carboxylic acid hexyl ester could completely blocks polymerase activity.

### Test example 2, enzyme activity test

The crude product of 3,4-dimethyl-2,5-dicarbonyl-2,5-dihydro-1H-pyrrole-1-carboxylic acid hexyl ester, pure product of 3,4-dimethyl-2,5-dicarbonyl-2,5-dihydro-1H-pyrrole-1-carboxylic acid hexyl ester and N-ethoxycarbonyl-2,3-disubstituted butenedimide were used to modify four hot-start enzymes (ABI, Bioline, Biog and domestic hot-start Taq enzyme), respectively. The addition amounts of each chemical modifier were 0.8x, 1.0x and 1.2x, respectively.

Add partially complementary primers to the qPCR system, And the hot-start Taq enzyme (ABI, Bioline, Biog and domestic hot start Taq enzyme) modified with crude product of 3,4-dimethyl-2,5-dicarbonyl-2,5-dihydro-1H-pyrrole-1-carboxylic acid hexyl ester, the hot-start Taq enzyme modified with pure product of 3,4-dimethyl-2,5-dicarbonyl-2,5-dihydro-1H-pyrrole-1-carboxylic acid hexyl ester are separately added to the system as the experimental group, with four hot-start Taq enzymes modified with N-ethoxycarbonyl-2,3-disubstituted butenedimide as the control group. Incubate at 37 degrees for 60 minutes. The preparation of hot start DNA polymerase modifications is shown in Table 1.

**Table 1. Preparation of DNA polymerase modifications**

| Group | Inhibitor stock solution concentration | Adding volume of inhibitor stock solution | Modify Buffer volume |
|---|---|---|---|
| control group | 105.7mg/ml | 60ul | 1.5ml |
| Experimental group added crude product (0.8x) | | 48u1 | |
| Experimental group added pure product (0.8x) | | 48ul | |
| Experimental group added crude product (1.0x) | | 60ul | |
| Experimental group added pure product (1.0x) | | 60ul | |
| Experimental group added crude product (1.2x) | | 72ul | |
| Experimental group added pure product (1.2x) | | 72ul | |

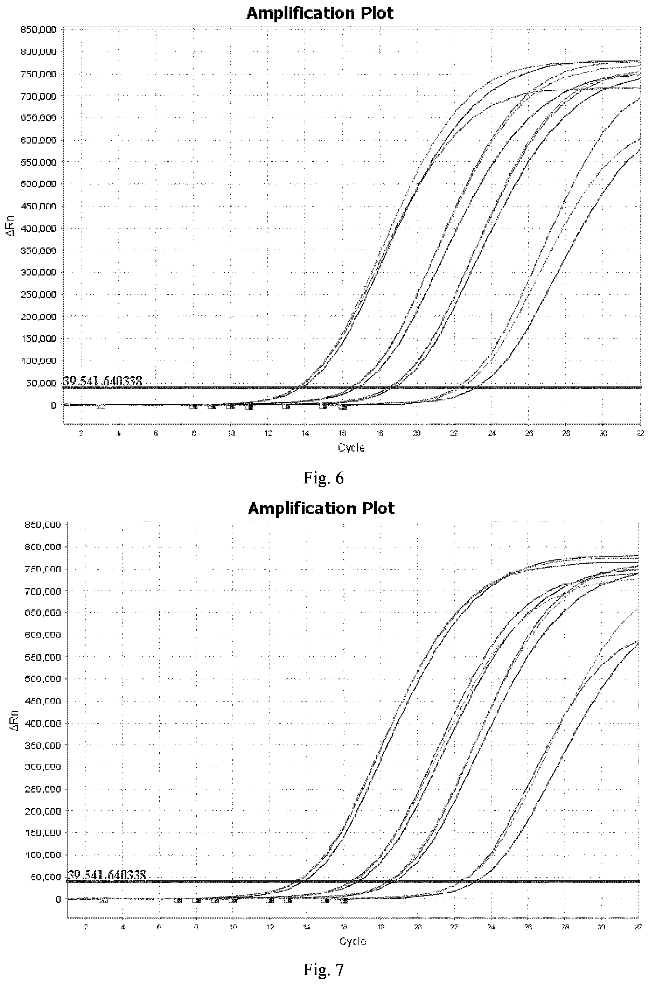

The test results of the 0.8x experimental group and the control group are shown in Figure 5; the test results of the 1.0x experimental group and the control group are shown in Figure 6; the test results of the 1.2x experimental group and the control group are shown in Figure 7.

In Figure 5, Figure 6 and Figure 7, the CT values from large to small are ABI, Bioline, Biog and domestic hot start Taq enzyme.

It can be seen from the original curve of qPCR curve that whether it is ABI, Bioline, Biog or domestic hot-start Taq enzyme, the hot-start Taq enzyme modified with pure product of 3,4-dimethyl-2,5-dicarbonyl-2,5-dihydro-1H-pyrrole-1-carboxylic acid hexyl ester has the highest CT value and higher fluorescence value, followed by hot-start Taq enzyme modified with crude product of 3,4-dimethyl-2,5-dicarbonyl-2,5-dihydro-1H-pyrrole-1-carboxylic acid hexyl ester. The hot-start Taq enzyme modified with N-ethoxycarbonyl-2,3-disubstituted butenedimide has the lowest CT value. It can be seen that modifying the hot-start Taq enzyme with pure product of 3,4-dimethyl-2,5-dicarbonyl-2,5-dihydro-1H-pyrrole-1-carboxylic acid hexyl ester can completely block the polymerase activity and its performance is significantly better than the hot-start Taq enzyme modified with crude product or N-ethoxycarbonyl-2,3-disubstituted butenedimide. It is calculated that the enzyme activity of the hot-start Taq enzyme modified with pure product of 3,4-dimethyl-2,5-dicarbonyl-2,5-dihydro-1H-pyrrole-1-carboxylic acid hexyl ester is 268 U/ul, while the activity of the hot-start Taq enzyme modified with N-ethoxycarbonyl-2,3-disubstituted butenedimide is only 221 U/µl, and the enzyme activity is increased by 21.8%.

**Test example three, accelerated stability test**

Accelerated stability tests were conducted using the hot-start Taq enzyme modified with pure product of 3,4-dimethyl-2,5-dicarbonyl-2,5-dihydro-1H-pyrrole-1-carboxylic acid hexyl ester, the hot-start Taq enzyme modified with crude product of 3,4-dimethyl-2,5-dicarbonyl-2,5-dihydro-1H-pyrrole-1-carboxylic acid hexyl ester, and the hot-start Taq enzyme modified with N-ethoxycarbonyl-2,3-disubstituted butenedimide, respectively.

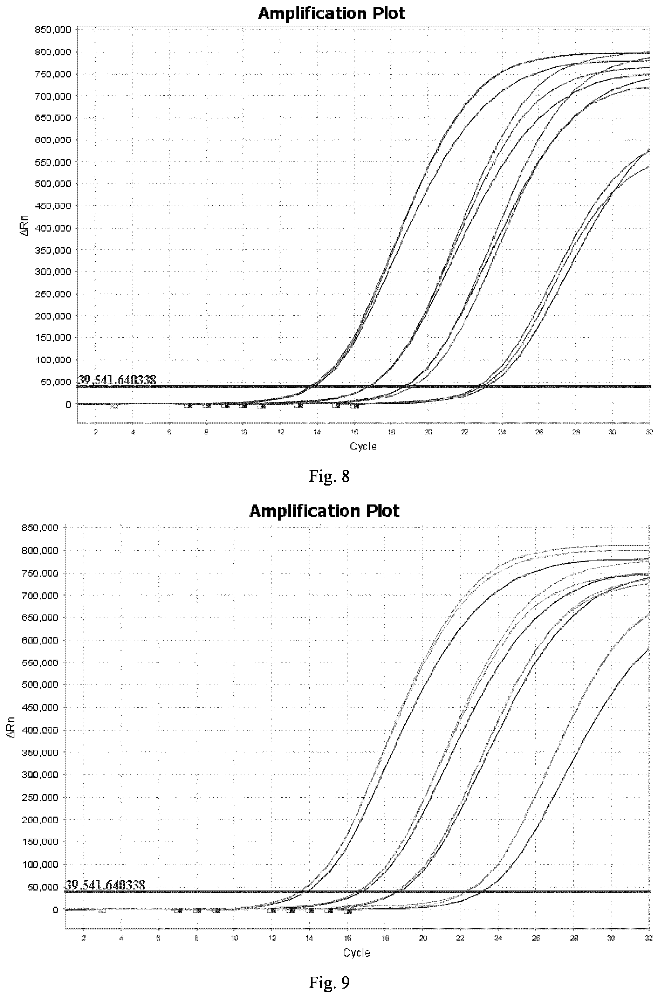

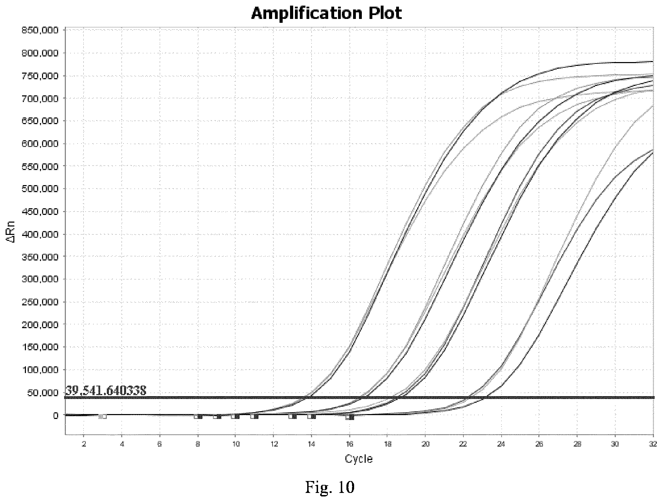

Figure 8 is a graph showing accelerated stability results of the 0.8x experimental group and the control group according to the embodiment of the present invention; Figure 9 is a graph showing accelerated stability results of the 1.0x experimental group and the control group according to the embodiment of the present invention; Figure 10 is a graph showing accelerated stability results of the 1.2x experimental group and the control group according to the embodiment of the present invention. In Figure 8, Figure 9 and Figure 10, the CT values from large to small are ABI, Bioline, Biog and domestic hot start Taq enzyme.

The results showed that after 7 days of storage at 37°C, it showed basically consistent stability using the hot-start Taq enzyme modified with pure product of 3,4-dimethyl-2,5-dicarbonyl-2,5-dihydro-1H-pyrrole-1-carboxylic acid hexyl ester, the hot-start Taq enzyme modified with crude product of 3,4-dimethyl-2,5-dicarbonyl-2,5-dihydro-1H-pyrrole-1-carboxylic acid hexyl ester, or the hot-start Taq enzyme modified with N-ethoxycarbonyl-2,3-disubstituted butenedimide.

It will be understood by one of ordinary skill in the art that the above embodiments are specific embodiments for realizing the present invention, and that various changes can be made thereto in form and detail in practical application without departing from the spirit and scope of the present invention.

## Claims

1. A Taq enzyme reversible modifier, wherein the structure of the Taq enzyme reversible modifier is shown in formula (I) 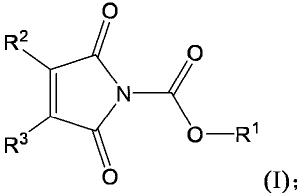
wherein, R¹ is C₃₋₆ alkyl;
R² and R³ are independently C₁₋₆ alkyl, respectively.

2. The Taq enzyme reversible modifier according to claim 1, wherein R¹ is unsubstituted C₃₋₆ linear alkyl;
and/or, R² and R³ are independently C₁₋₄ alkyl, respectively.

3. The Taq enzyme reversible modifier according to claim 1, wherein R¹ is n-propyl, n-butyl, n-pentyl or n-hexyl;
and/or, R² and R³ are independently methyl, ethyl, n-propyl, isopropyl, n-butyl, sec-butyl, isobutyl or tert-butyl, respectively.

4. The Taq enzyme reversible modifier according to claim 1, wherein R¹ is n-hexyl.

5. The Taq enzyme reversible modifier according to claim 1, wherein the structure of the Taq enzyme reversible modifier is shown in formula (II):

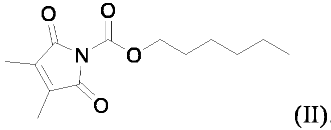

6. The Taq enzyme reversible modifier according to claim 5, wherein the Taq enzyme reversible modifier is obtained by purifying the crude product of 3,4-dimethyl-2,5-dicarbonyl-2,5-dihydro-1H-pyrrole-1-carboxylic acid hexyl ester using high performance liquid chromatography.

7. A chemically modified Taq enzyme, wherein the chemically modified Taq enzyme is modified by the Taq enzyme reversible modifier described in any one of claims 1 to 6.

8. A PCR kit, wherein the PCR kit comprises the chemically modified Taq enzyme according to claim 7.

9. A method for preparing the chemically modified Taq enzyme according to claim 7, wherein the method includes the step of mixing wild-type Taq enzyme and the Taq enzyme reversible modifier according to any one of claims 1 to 6.

10. The method according to claim 9, wherein the mixing is performed in an oscillator, and the mixing time is 15 to 30 seconds.

11. Use of a compound of formula (1) for:
(i) preparing Taq enzyme reversible modifier;
(ii) modifying Taq enzyme reversibly.
